# EUROPEAN PATENT SPECIFICATION

(11) **EP 2 836 475 B1**
(45) Date of publication and mention of the grant of the patent: **26.05.2021**
(21) Application number: 13721435.9
(22) Date of filing: 11.04.2013
(51) Int. Cl.: C07C 51/377

(54) **PURIFICATION OF BIO-BASED ACRYLIC ACID TO CRUDE AND GLACIAL ACRYLIC ACID**
REINIGUNG VON BIOBASIERTER ACRYLSÄURE ZU ROH- UND KALTACRYLSÄURE
PURIFICATION D'ACIDE ACRYLIQUE D'ORIGINE BIOLOGIQUE POUR OBTENIR DE L'ACIDE ACRYLIQUE GLACIAL ET BRUT

(30) Priority: 11.04.2012 US 201261623054 P; 06.02.2013 US 201313760505; 15.03.2013 US 201313835187; 15.03.2013 US 201313838823
(43) Date of publication of application: 18.02.2015
(73) Proprietor: The Procter & Gamble Company, Cincinnati, OH 45202 (US)
(72) Inventor: GODLEWSKI, Jane, Ellen, Cincinnati, OH 45202 (US); LINGOES, Janette, Villalobos, Cincinnati, OH 45202 (US); VELASQUEZ, Juan, Esteban, Cincinnati, OH 45202 (US); COLLIAS, Dimitris, Ioannis, Cincinnati, OH 45202 (US)
(74) Representative: P&G Patent Germany
(86) International application number: PCT/US2013/036158
(87) International publication number: WO 2013/155292

(56) References cited:
- WO-A1-2008/023039

## Description

### FIELD OF THE INVENTION

The present invention generally according to the appended claims relates to a glacial acrylic acid composition comprising at least 98 wt% acrylic acid, and wherein a portion of the remaining impurities in said glacial acrylic acid composition is propanoic acid; wherein said glacial acrylic acid composition has a bio-based content greater than 3%; and to a process for producing that glacial acrylic acid.

### BACKGROUND OF THE INVENTION

Acrylic acid or acrylate has a variety of industrial uses, typically consumed in the form of polymers. In turn, these polymers are commonly used in the manufacture of, among other things, adhesives, binders, coatings, paints, polishes, detergents, flocculants, dispersants, thixotropic agents, sequestrants, and superabsorbent polymers, which are used in disposable absorbent articles including diapers and hygienic products, for example. Acrylic acid is commonly made from petroleum sources. For example, acrylic acid has long been prepared by catalytic oxidation of propylene. These and other methods of making acrylic acid from petroleum sources are described in the Kirk-Othmer Encyclopedia of Chemical Technology, Vol. 1, pgs. 342 - 369 (5th Ed., John Wiley & Sons, Inc., 2004). Petroleum-based acrylic acid contributes to greenhouse emissions due to its high petroleum derived carbon content. Furthermore, petroleum is a non-renewable material, as it takes hundreds of thousands of years to form naturally and only a short time to consume. As petrochemical resources become increasingly scarce, more expensive, and subject to regulations for CO₂ emissions, there exists a growing need for bio-based acrylic acid or acrylate that can serve as an alternative to petroleum-based acrylic acid or acrylate. Many attempts have been made over the last 40 to 50 years to make bio-based acrylic acid or acrylate from non petroleum sources, such as lactic acid (also known as 2-hydroxypropionic acid), 3-hydroxypropionic acid, glycerin, carbon monoxide and ethylene oxide, carbon dioxide and ethylene, and crotonic acid.

Petroleum-based acrylic acid is produced by the heterogeneously-catalyzed gas-phase oxidation of propylene with the use of molecular oxygen. Typical side products in this process are carbonyl compounds, such as, benzaldehyde, furfurals, propionaldehyde, etc., and acids or anhydrides, such as, formic acid, propanoic acid, acetic acid, and maleic acid, or maleic anhydride. The typical composition in wt% of a reaction gas coming out of the process is (see U.S. Patent No. 7,179,875 (issued in 2007)): acrylic acid up to 30%, steam up to 30%, carbon oxides up to 15%, nitrogen up to 90%, oxygen up to 10%, propylene up to 1%, acrolein up to 2%, propane up to 2%, formic acid up to 1%, acetic acid up to 2%, propionic acid up to 2%, aldehydes up to 3%, and maleic anhydride up to 0.5%.

Depending on the end use, there are two purity levels of acrylic acid: crude acrylic acid (also called technical grade acrylic acid) and glacial acrylic acid. Crude acrylic acid has a typical minimum overall purity level of 94% and is used to make acrylic esters for paint, adhesive, textile, paper, leather, fiber, and plastic additive applications. Glacial acrylic acid has a typical overall purity level ranging from 98% to 99.7% and is used to make polyacrylic acid for superabsorbent polymer (SAP; in disposable diapers, training pants, adult incontinence undergarments, etc.), paper and water treatment, and detergent co-builder applications. The levels of the impurities need to be as low as possible in glacial acrylic acid to allow for a high-degree of polymerization to acrylic acid polymers (PAA) and avoid adverse effects of side products in applications. For example, aldehydes hinder the polymerization and also lead to discoloration of the polymerized acrylic acid; maleic anhydride forms undesirable copolymers which have a detriment to the polymer properties; and carboxylic acids, that do not participate in the polymerization, might affect the final odor of PAA or SAP or provide adverse effects in the use of the products, e.g. skin irritation when the SAP contains formic acid, or odor when the SAP contains acetic acid or propionic acid. To remove or reduce the amounts of side products from petroleum-based acrylic acid and produce either petroleum-based crude acrylic acid or petroleum-based glacial acrylic acid, multistage distillations and / or extraction and / or crystallizations steps were employed in the prior art (e.g. see U.S. Patent Nos. 5,705,688 (issued in 1998), and 6,541,665 (issued in 2003)).

Bio-based acrylic acid, produced from renewable feedstocks or intermediate chemicals (e.g. lactic acid or lactate, glycerin, 3-hydroxypropionic acid or its ester, etc.), has different impurity profiles and levels than petroleum-based acrylic acid. For example, when lactic acid is used as the intermediate chemical, the major impurities are acetaldehyde, acetic acid, lactic acid, and propanoic acid. Nevertheless, the minimum overall purity levels of bio-based crude acrylic acid and bio-based glacial acrylic acid required for the final applications from bio-based acrylic acid are expected to be the same as those in petroleum-based acrylic acid, i.e., 94% and 98%, respectively WO 2008/023039 describe a process for preparing crude acrylic acid.

Accordingly, there is a need for commercially viable processes to purify bio-based acrylic acid produced from the dehydration of hydroxypropionic acid, hydroxypropionic acid derivatives, or mixtures thereof, to crude or glacial acrylic acid.

### SUMMARY OF THE INVENTION

The present invention is defined in present claims 1 to 13.

### DETAILED DESCRIPTION OF THE INVENTION

### I Definitions

As used herein, the term "distilled acrylic acid" refers to a composition of acrylic acid with content of acrylic acid lower than 94 wt%.

As used herein, the term "crude acrylic acid" refers to a composition of acrylic acid with content of acrylic acid between 94 wt% and 98 wt%.

As used herein, the term "glacial acrylic acid" refers to a composition of acrylic acid with content of acrylic acid at least 98 wt%.

As used herein, the term "bio-based" material refers to a renewable material.

As used herein, the term "renewable material" refers to a material that is produced from a renewable resource.

As used herein, the term "renewable resource" refers to a resource that is produced via a natural process at a rate comparable to its rate of consumption (e.g., within a 100 year time frame). The resource can be replenished naturally, or via agricultural techniques. Non limiting examples of renewable resources include plants (e.g., sugar cane, beets, corn, potatoes, citrus fruit, woody plants, lignocellulose, hemicellulose, cellulosic waste), animals, fish, bacteria, fungi, and forestry products. These resources can be naturally occurring, hybrids, or genetically engineered organisms. Natural resources, such as crude oil, coal, natural gas, and peat, which take longer than 100 years to form, are not considered renewable resources. Because at least part of the material of the invention is derived from a renewable resource, which can sequester carbon dioxide, use of the material can reduce global warming potential and fossil fuel consumption.

As used herein, the term "bio-based content" refers to the amount of carbon from a renewable resource in a material as a percent of the weight (mass) of the total organic carbon in the material, as determined by ASTM D6866-10, Method B.

As used herein, the term "petroleum-based" material refers to a material that is produced from fossil material, such as petroleum, natural gas, coal, etc.

As used herein, the term "condensed phosphate" refers to any salts containing one or several P-O-P bonds generated by corner sharing of PO₄ tetrahedra.

As used herein, the term "cyclophosphate" refers to any cyclic condensed phosphate constituted of two or more corner-sharing PO₄ tetrahedra.

As used herein, the term "monophosphate" or "orthophosphate" refers to any salt whose anionic entity, [PO₄]³⁻, is composed of four oxygen atoms arranged in an almost regular tetrahedral array about a central phosphorus atom.

As used herein, the term "oligophosphate" refers to any polyphosphates that contain five or less PO₄ units.

As used herein, the term "polyphosphate" refers to any condensed phosphates containing linear P-O-P linkages by corner sharing of PO₄ tetrahedra leading to the formation of finite chains.

As used herein, the term "ultraphosphate" refers to any condensed phosphate where at least two PO₄ tetrahedra of the anionic entity share three of their corners with the adjacent ones.

As used herein, the term "cation" refers to any atom or group of covalently-bonded atoms having a positive charge.

As used herein, the term "monovalent cation" refers to any cation with a positive charge of +1.

As used herein, the term "polyvalent cation" refers to any cation with a positive charge equal or greater than +2.

As used herein, the term "anion" refers to any atom or group of covalently-bonded atoms having a negative charge.

As used herein, the term "heteropolyanion" refers to any anion with covalently bonded XOₚ and YOᵣ polyhedra, and thus includes X-O-Y and possibly X-O-X and Y-O-Y bonds, wherein X and Y represent any atoms, and wherein p and r are any positive integers.

As used herein, the term "heteropolyphosphate" refers to any heteropolyanion, wherein X represents phosphorus (P) and Y represents any other atom.

As used herein, the term "phosphate adduct" refers to any compound with one or more phosphate anions and one or more non-phosphate anions that are not covalently linked.

As used herein, the terms "LA" refers to lactic acid, "AA" refers to acrylic acid, "AcH" refers to acetaldehyde, and "PA" refers to propionic acid.

As used herein, the term "particle span" refers to a statistical representation of a given particle sample and is equal to (*D_{v,0.90}* - *D_{v,0.10}*)/*D_{v,0.50}*. The term "median particle size" or *D_{v,0.50}* refers to the diameter of a particle below which 50% of the total volume of particles lies. Further, *D_{v,0.10}* refers to the particle size that separates the particle sample at the 10% by volume fraction and *D*_{*v*,*0.90*}, is the particle size that separates the particle sample at the 90% by volume fraction.

As used herein, the term "conversion" in % is defined as [hydroxypropionic acid, hydroxypropionic acid derivatives, or mixtures thereof flow rate in (mol/min) - hydroxypropionic acid, hydroxypropionic acid derivatives, or mixtures thereof flow rate out (mol/min)] / [hydroxypropionic acid, hydroxypropionic acid derivatives, or mixtures thereof flow rate in (mol/min)] *100. For the purposes of this invention, the term "conversion" means molar conversion, unless otherwise noted.

As used herein, the term "yield" in % is defined as [product flow rate out (mol/min) / hydroxypropionic acid, hydroxypropionic acid derivatives, or mixtures thereof flow rate in (mol/min)]*100. For the purposes of this invention, the term "yield" means molar yield, unless otherwise noted.

As used herein, the term "selectivity" in % is defined as [Yield / Conversion]^{∗}100. For the purposes of this invention, the term "selectivity" means molar selectivity, unless otherwise noted.

As used herein, the term "total flow rate out" in mol/min and for hydroxypropionic acid is defined as: (2/3)^{∗}[C2 flow rate out (mol/min)] + [C3 flow rate out (mol/min)] + (2/3)^{∗}[acetaldehyde flow rate out (mol/min)] + (4/3)^{∗}[C4 flow rate out (mol/min)] + [hydroxypropionic acid flow rate out (mol/min)] + [pyruvic acid flow rate out (mol/min)] + (2/3)^{∗}[acetic acid flow rate out (mol/min)] + [1,2-propanediol flow rate out (mol/min)] + [propionic acid flow rate out (mol/min)] + [acrylic acid flow rate out (mol/min)] + (5/3)^{∗}[2,3-pentanedione flow rate out (mol/min)] + (1/3)^{∗}[carbon monoxide flow rate out (mol/min)] + (1/3)^{∗}[carbon dioxide flow rate out (mol/min)]. If a hydroxypropionic acid derivative is used instead of hydroxypropionic acid, the above formula needs to be adjusted according to the number of carbon atoms in the hydroxypropionic acid derivative.

As used herein, the term "C2" means ethane and ethylene.

As used herein, the term "C3" means propane and propylene.

As used herein, the term "C4" means butane and butenes.

As used herein, the term "total molar balance" or "TMB" in % is defined as [total flow rate out (mol/min) / hydroxypropionic acid, hydroxypropionic acid derivatives, or mixtures thereof flow rate in (mol/min)]^{∗}100.

As used herein, the term "the acrylic acid yield was corrected for TMB" is defined as [acrylic acid yield / total molar balance]^{∗}100, to account for slightly higher flows in the reactor.

As used herein, the term "Gas Hourly Space Velocity" or "GHSV" in h⁻¹ is defined as 60 x [Total gas flow rate (mL/min) / catalyst bed volume (mL)]. The total gas flow rate is calculated under Standard Temperature and Pressure conditions (STP; 0°C and 1 atm).

As used herein, the term "Liquid Hourly Space Velocity" or "LHSV" in h⁻¹ is defined as 60 x [Total liquid flow rate (mL/min) / catalyst bed volume (mL)].

### II Purification Processes

Unexpectedly it has been found that, some or all of the processes of extraction, drying, distilling, cooling, partial melting, and decanting can be used to produce crude and glacial acrylic acid produced from bio-based acrylic acid. Although the impurities that are present in bio-based acrylic acid are different than those present in petroleum-based acrylic acid, the same processes that are used to purify the petroleum-based acrylic acid can be used to purify bio-based acrylic acid to crude or glacial purity levels.

In one embodiment, a glacial acrylic acid composition is provided comprising at least about 98 wt% acrylic acid, and wherein a portion of the remaining impurities in the glacial acrylic acid composition is hydroxypropionic acid, hydroxypropionic acid derivatives, or mixtures thereof.

A crude acrylic acid composition is provided comprising between 94 wt% and 98 wt% acrylic acid, and wherein a portion of the remaining impurities in the glacial acrylic acid composition is hydroxypropionic acid, hydroxypropionic acid derivatives, or mixtures thereof.

Hydroxypropionic acid can be 3-hydroxypropionic acid, 2-hydroxypropionic acid (also called, lactic acid), 2-methyl hydroxypropionic acid, or mixtures thereof. Derivatives of hydroxypropionic acid can be metal or ammonium salts of hydroxypropionic acid, alkyl esters of hydroxypropionic acid, alkyl esters of 2-methyl hydroxypropionic acid, cyclic di-esters of hydroxypropionic acid, hydroxypropionic acid anhydride, or a mixture thereof. Non-limiting examples of metal salts of hydroxypropionic acid are sodium hydroxypropionate, potassium hydroxypropionate, and calcium hydroxypropionate. Non-limiting examples of alkyl esters of hydroxypropionic acid are methyl hydroxypropionate, ethyl hydroxypropionate, butyl hydroxypropionate, 2-ethylhexyl hydroxypropionate, or mixtures thereof. A non-limiting example of cyclic di-esters of hydroxypropionic acid is dilactide.

In one embodiment, the hydroxypropionic acid is lactic acid or 2-methyl lactic acid. In another embodiment, the hydroxypropionic acid is lactic acid. Lactic acid can be L-lactic acid, D-lactic acid, or mixtures thereof. In one embodiment, the hydroxypropionic acid, hydroxypropionic acid derivatives, or mixtures thereof in the impurities in the glacial acrylic acid composition are lactic acid, lactic acid derivatives, or mixtures thereof. In another embodiment, the hydroxypropionic acid, hydroxypropionic acid derivatives, or mixtures thereof in the impurities in the crude acrylic acid composition are lactic acid, lactic acid derivatives, or mixtures thereof.

In one embodiment, the concentration of the hydroxypropionic acid, hydroxypropionic acid derivatives, or mixtures thereof in the remaining impurities of the glacial acrylic acid composition is less than 2 wt%, based on the total amount of the glacial acrylic acid composition. In another embodiment, the hydroxypropionic acid, hydroxypropionic acid derivatives, or mixtures thereof in the remaining impurities of the glacial acrylic acid composition is less than 1 wt%, based on the total amount of the glacial acrylic acid composition. In another embodiment, the hydroxypropionic acid, hydroxypropionic acid derivatives, or mixtures thereof in the remaining impurities of the glacial acrylic acid composition is less than 400 ppm, based on the total amount of the glacial acrylic acid composition.

In the present invention, the bio-based content of the glacial acrylic acid is greater than 3%. In another embodiment, the bio-based content of the glacial acrylic acid is greater than 30%. In yet another embodiment, the bio-based content of the glacial acrylic acid is greater than 90%. The bio-based content of the crude acrylic acid is greater than 3%. The the bio-based content of the crude acrylic acid is greater than 30%.

The bio-based content of the crude acrylic acid is greater than 90%.

The glacial or crude acrylic acid composition can be made from an aqueous solution of acrylic acid produced from renewable resources or materials and fed into the purification process to produce crude acrylic acid or glacial acrylic acid. Non-limiting examples of renewable resources or materials for the production of the aqueous solution of acrylic acid are hydroxypropionic acid, hydroxypropionic acid derivatives, or mixtures thereof; glycerin; carbon monoxide and ethylene oxide; carbon dioxide and ethylene; and crotonic acid. In one embodiment, the renewable resources or materials are hydroxypropionic acid, hydroxypropionic acid derivatives, or mixtures thereof. In another embodiment, the renewable resources or materials are lactic acid, lactic acid derivatives, or mixtures thereof. In yet another embodiment, the renewable resource or material is lactic acid.

In one embodiment, the aqueous solution of acrylic acid comprises: 1) acrylic acid; 2) lactic acid, lactic acid derivatives, or mixtures thereof, and is essentially free of maleic anhydride, furfural, and formic acid. In another embodiment, the aqueous solution of acrylic acid has from 4 wt% to 80 wt% acrylic acid. In another embodiment, the aqueous solution of acrylic acid has from 4 wt% to 40 wt% acrylic acid. In yet another embodiment, the aqueous solution of acrylic acid has from 5 wt% to 25 wt% acrylic acid. In another embodiment, the aqueous solution of acrylic acid has from 8 wt% to 16 wt% acrylic acid.

In one embodiment, the aqueous solution of acrylic acid has from 0.001 wt% to 50 wt% lactic acid, lactic acid derivatives, or mixtures thereof. In another embodiment, the aqueous solution of acrylic acid has from 0.001 wt% to 20 wt% % lactic acid, lactic acid derivatives, or mixtures thereof. In yet another embodiment, the aqueous solution of acrylic acid has 6 wt% % lactic acid, lactic acid derivatives, or mixtures thereof.

In one embodiment, the aqueous solution of acrylic acid has from 8 wt% to 16 wt% acrylic acid and from 0.1 wt% to 10 wt% lactic acid, lactic acid derivatives, or mixtures thereof, and wherein said aqueous solution of acrylic acid is essentially free of maleic anhydride, furfural, and formic acid. Non-limiting examples of impurities that can be present in the aqueous solution of acrylic acid are acetaldehyde, acetic acid, and propanoic acid.

The aqueous solution of acrylic acid can be extracted with a solvent to produce an extract. In one embodiment, the solvent is selected from the group consisting of ethyl acetate, isobutyl acetate, methyl acetate, toluene, dimethyl phthalate, hexane, pentane, diphenyl ether, ethyl hexanoic acid, N-methylpyrrolidone, C6 to C10 paraffin fractions, and mixtures thereof. In another embodiment, the extraction solvent is ethyl acetate. In one embodiment, the extraction solvent can form an azeotrope with water.

The solvent comprises at least one polymerization inhibitor. Non-limiting examples of polymerization inhibitors are phenothiazine and 4-methoxy phenol. In another embodiment, the glacial acrylic acid comprises from about 200 ppm to about 400 ppm 4-methoxyphenol. In another embodiment, the polymerization inhibitor is added to the aqueous solution of acrylic acid before the extracting step.

After the extraction, the extract can be dried to produce a dried extract. The drying can be achieved with a variety of methods, such as, and not by way of limitation, distillation and sorption. In one embodiment, the drying is performed by azeotropic distillation. In another embodiment, the sorption is performed on a solid powder. In yet another embodiment, the solid powder is selected from the group consisting of magnesium sulfate, sodium sulfate, calcium sulfate, molecular sieves, metal hydrides, reactive metals, and mixtures thereof. In yet another embodiment, the sorption is performed with sodium sulfate and is followed by filtration to produce a dried filtrate.

The dried extract or dried filtrate can be further processed by distillation to produce a distilled acrylic acid composition. In one embodiment, the distillation is vacuum distillation at about 70 mm Hg and about 40°C to produce a distilled crude acrylic acid composition, and is followed by a fractional distillation at about 40 mm Hg and collecting fractions from 59°C to 62°C to produce the distilled acrylic acid composition.

In one embodiment, cooling of the distilled acrylic acid composition to a temperature from -21°C to 14°C produces crystals of acrylic acid; partially melting the crystals of acrylic acid produces a liquid / solid mixture; decanting the liquid / solid mixture produces a purified acrylic acid solid composition; fully melting the purified acrylic acid solid composition produces a purified acrylic acid liquid composition; and determining acrylic acid purity of the purified acrylic acid liquid composition, and if the purity is less than 98 wt% acrylic acid, repeating said cooling, partially melting, decanting, and fully melting steps on the purified acrylic acid liquid composition until a purity of 98 wt% acrylic acid is achieved and a glacial acrylic acid composition is produced.

In yet another embodiment, cooling of the distilled acrylic acid composition to a temperature from -21°C to 14°C produces crystals of acrylic acid; partially melting the crystals of acrylic acid produces a liquid / solid mixture; decanting the liquid / solid mixture produces a purified acrylic acid solid composition; fully melting the purified acrylic acid solid composition produces a purified acrylic acid liquid composition; and determining acrylic acid purity of the purified acrylic acid liquid composition, and if the purity is less than 99 wt% acrylic acid, repeating said cooling, partially melting, decanting, and fully melting steps on the purified acrylic acid liquid composition until a purity of 99 wt% acrylic acid is achieved and a glacial acrylic acid composition is produced.

In one embodiment, the distilled acrylic acid composition is cooled to a temperature from 0°C to 5°C to produce crystals of acrylic acid.

In one embodiment of the present invention, the glacial acrylic acid composition is produced by the steps comprising: a) providing an aqueous solution of acrylic acid comprising 1) acrylic acid and 2) lactic acid, lactic acid derivatives, or mixtures thereof, and wherein said aqueous solution of acrylic acid is essentially free of maleic anhydride, furfural, and formic acid; b) extracting said aqueous solution of acrylic acid with a solvent to produce an extract; c) drying said extract to produce a dried extract; d) distilling said dried extract to produce crude acrylic acid; e) cooling said crude acrylic acid to a temperature from -21 °C to 14°C to produce crystals of acrylic acid; f) partially melting said crystals of acrylic acid to produce a liquid / solid mixture; g) decanting said liquid / solid mixture to produce a acrylic acid solid composition; h) fully melting said purified acrylic acid solid composition to produce a purified acrylic acid composition; and i) determining the acrylic acid purity of said purified acrylic acid liquid composition and if the purity is less than 98 wt% acrylic acid repeating said cooling, partially melting, decanting, and fully melting steps on the purified acrylic acid liquid composition until a purity of 98 wt% is achieved to produce glacial acrylic acid composition.

### III Catalysts for the Conversion of Hydroxypropionic Acid or its Derivatives to Acrylic Acid or its Derivatives

The catalyst includes: (a) monohydrogen monophosphate and dihydrogen monophosphate anions described by formulae (I) and (II):

[HPO₄]²⁻ (I),

[H₂PO₄]⁻ (II),

and (b) at least two different cations, wherein the catalyst is essentially neutrally charged; and further, wherein the molar ratio of said monohydrogen monophosphate anion to said dihydrogen monophosphate anion in the catalyst is between about 0.1 and about 10. In another embodiment, the molar ratio of monohydrogen monophosphate anion to dihydrogen monophosphate anion is between about 0.2 and about 5. In yet another embodiment, the molar ratio of monohydrogen monophosphate anion to dihydrogen monophosphate anion is about 1.

The catalyst includes the monophosphate salts described by the formulae (III) and (IV):

M^{II}HPO₄ (III),

M^{I}H₂PO₄ (IV),

and
wherein M^{I} is a monovalent cation and M^{II} is a divalent cation. In another embodiment, the molar ratio of M^{II}HPO₄ to M^{I}H₂PO₄ is between about 0.1 and about 10. In another embodiment, the molar ratio of M^{II}HPO₄ to M^{I}H₂PO₄ is between about 0.2 and about 5. In yet another embodiment, the molar ratio of M^{II}HPO₄ to M^{I}H₂PO₄ is about 1.

The catalyst includes a monophosphate salt described by the formula (V):

M^{II}_{2-α}M^{I}_{α}H_{α} (HPO₄)₂ (V),

wherein M^{I} is a monovalent cation and M^{II} is a divalent cation; and wherein α is greater than about 0.2 and smaller than about 1.8. In another embodiment of the present invention, α is about 1.

The monohydrogen monophosphate anion described by formula (I) is substituted by one or more phosphate anions described by the formula [H_{(1-β)}P_{(1+β)}O_{(4+3β)}]^{2(1+β)-}, wherein β is greater or equal to zero and less or equal to 1.

The dihydrogen monophosphate anion described by formula (II) is substituted by one or more phosphate anions described by the formula [H_{2(1-β})PO_{(4-β)}]⁻, wherein β is greater or equal to zero and less or equal to 1.

The catalyst comprises: (a) at least one condensed phosphate anion selected from the group consisting of formulae (VI), (VII), and (VIII),

[PₙO₃ₙ₊₁]⁽ⁿ⁺²⁾⁻ (VI)

[PₙO₃ₙ]ⁿ⁻ (VII)

[P₍₂ₘ₊ₙ₎O₍₅ₘ₊₃ₙ₎]ⁿ⁻ (VIII)

wherein n is at least 2 and m is at least 1, and (b) at least two different cations, wherein the catalyst is essentially neutrally charged, and further, wherein the molar ratio of phosphorus to the at least two different cations is between about 0.7 and about 1.7.

The anions defined by formulae (VI), (VII), and (VIII) are also referred to as polyphosphates (or oligophosphates), cyclophosphates, and ultraphosphates, respectively.

The catalyst comprises: (a) at least one condensed phosphate anion selected from the group consisting of formulae (VI) and (VII),

[PₙO₃ₙ₊₁]⁽ⁿ⁺²⁾⁻ (VI)

[PₙO₃ₙ]ⁿ⁻ (VII)

wherein n is at least 2, and (b) at least two different cations, wherein the catalyst is essentially neutrally charged, and further, wherein the molar ratio of phosphorus to the at least two different cations is between about 0.7 and about 1.7.

The molar ratio of phosphorus to the cations in the catalyst is between about 0.7 and about 1.7; in another embodiment, the molar ratio of phosphorus to the cations in the catalyst is between about 0.8 and about 1.3; and in yet another embodiment, the molar ratio of phosphorus to the cations in the catalyst is about 1.

The at least two different cations comprise (a) at least one monovalent cation, and (b) at least one polyvalent cation. In another embodiment, the molar ratio of the monovalent cations to the polyvalent cations is between about 0.1 and about 10. The molar ratio of the monovalent cations to the polyvalent cations is between about 0.5 and about 5. In a further embodiment of the present invention, the molar ratio of the monovalent cations to the polyvalent cations is about 1.

The polyvalent cation is selected from the group consisting of divalent cations, trivalent cations, tetravalent cations, pentavalent cations, and mixtures thereof. Non-limiting examples of monovalent cations are H⁺, Li⁺, Na⁺, K⁺, Rb⁺, Cs⁺, Ag⁺, Rb⁺, Tl⁺, and mixtures thereof. The monovalent cation is selected from the group consisting of Li⁺, Na⁺, K⁺, Rb⁺, Cs⁺, and mixtures thereof; The monovalent cation is Na⁺ or K⁺; and the the monovalent cation is K⁺. Non-limiting examples of polyvalent cations are cations of the alkaline earth metals (i.e., Be, Mg, Ca, Sr, Ba, and Ra), transition metals (e.g. Y, Ti, Zr, V, Nb, Cr, Mo, Mn, Re, Fe, Ru, Co, Rh, Ni, Pd, Pt, Cu, Ag, and Au), poor metals (e.g. Zn, Ga, Si, Ge, B, Al, In, Sb, Sn, Bi, and Pb), lanthanides (e.g. La and Ce), and actinides (e.g. Ac and Th). The polyvalent cation is selected from the group consisting of Be²⁺, Mg²⁺, Ca²⁺, Sr²⁺, Ba²⁺, Mn²⁺, Fe²⁺, Co²⁺, Ni²⁺, Cu²⁺, Zn²⁺, Cd²⁺, Sn²⁺, Pb²⁺, Ti³⁺, Cr³⁺, Mn³⁺, Fe³⁺, Al³⁺, Ga³⁺, Y³⁺, In³⁺, Sb³⁺, Bi³⁺, Si⁴⁺, Ti⁴⁺, V⁴⁺, Ge⁴⁺, Mo⁴⁺, Pt⁴⁺, V⁵⁺, Nb⁵⁺, Sb⁵⁺, and mixtures thereof. The polyvalent cation is selected from the group consisting of Ca²⁺, Ba²⁺, Cu²⁺, Mn²⁺, Mn³⁺, and mixtures thereof. The polyvalent cation is selected from the group consisting of Ca²⁺, Ba²⁺, Mn³⁺, and mixtures thereof; the polyvalent cation is Ba²⁺.

The catalyst can include cations: (a) H⁺, Li⁺, Na⁺, K⁺, Rb⁺, Cs⁺, or mixtures thereof; and (b) Be²⁺, Mg²⁺, Ca²⁺, Sr²⁺, Ba²⁺, Mn²⁺, Fe²⁺, Co²⁺, Ni²⁺, Cu²⁺, Zn²⁺, Cd²⁺, Sn²⁺, Pb²⁺, Ti³⁺, Cr³⁺, Mn³⁺, Fe³⁺, Al³⁺, Ga³⁺, Y³⁺, In³⁺, Sb³⁺, Bi³⁺, Si⁴⁺, Ti⁴⁺, V⁴⁺, Ge⁴⁺, Mo⁴⁺, Pt⁴⁺, V⁵⁺ , Nb⁵⁺, Sb⁵⁺, or mixtures thereof. In one embodiment the catalyst comprises Li⁺, Na⁺, or K⁺ as monovalent cation, and Ca²⁺, Ba²⁺, or Mn³⁺ as polyvalent cation; in another embodiment, the catalyst comprises Na⁺ or K⁺ as monovalent cation, and Ca²⁺ or Ba²⁺ as polyvalent cation; and in yet another embodiment, the catalyst comprises K⁺ as the monovalent cation and Ba²⁺ as the polyvalent cation.

The catalyst comprises Ba₂₋ₓ₋ₛK₂ₓH₂ₛP₂O₇ and (KPO₃)ₙ, wherein x and s are greater or equal to 0 and less than about 0.5 and n is a positive integer. The catalyst comprises Ca₂₋ₓ₋ₛK₂ₓH₂ₛP₂O₇ and (KPO₃)ₙ, wherein x and s are greater or equal to 0 and less than about 0.5 and n is at least 2. The catalyst comprises Mn₁₋ₓ₋ₛK₁₊₃ₓH₃ₛP₂O₇ or Mn₁₋ₓ₋ₛK₂₊₂ₓH₂ₛP₂O₇ and (KPO₃)ₙ wherein x and s are greater or equal to 0 and less than about 0.5 and n is at least 2. The catalyst comprises any blend of Ba₂₋ₓ₋ₛK₂ₓH₂ₛP₂O₇, Ca₂₋ₓ₋ₛK₂ₓH₂ₛP₂O₇, Mn₁₋ₓ₋ₛK₁₊₃ₓH₃ₛP₂O₇ or Mn₁₋ₓ₋ₛK₂₊₂ₓH₂ₛP₂O₇; and (KPO₃)ₙ, wherein x and s are greater or equal to 0 and less than about 0.5 and n is at least 2.

The catalyst comprises: (a) at least two different condensed phosphate anions selected from the group consisting of formulae (VI), (VII), and (VIII),

[PₙO₃ₙ₊₁]⁽ⁿ⁺²⁾⁻ (VI)

[PₙO₃ₙ]ⁿ⁻ (VII)

[P₍₂ₘ₊ₙ₎O₍₅ₘ₊₃ₙ₎]ⁿ⁻ (VIII)

wherein n is at least 2 and m is at least 1, and (b) one cation, wherein the catalyst is essentially neutrally charged, and further, wherein the molar ratio of phosphorus to the cation is between about 0.5 and about 4.0. The molar ratio of phosphorus to the cation is between about t/2 and about t, wherein t is the charge of the cation.

The catalyst can include an inert support that is constructed of a material comprising silicates, aluminates, carbons, metal oxides, and mixtures thereof. Alternatively, the carrier is inert relative to the reaction mixture expected to contact the catalyst. In the context of the reactions expressly described herein, in one embodiment the carrier is a low surface area silica or zirconia. When present, the carrier represents an amount of about 5 wt% to about 98 wt%, based on the total weight of the catalyst. Generally, a catalyst that includes an inert support can be made by one of two exemplary methods: impregnation or co-precipitation. In the impregnation method, a suspension of the solid inert support is treated with a solution of a pre-catalyst, and the resulting material is then activated under conditions that will convert the pre-catalyst to a more active state. In the co-precipitation method, a homogenous solution of the catalyst ingredients is precipitated by the addition of additional ingredients.

The catalyst can be sulfate salts; phosphate salts; mixtures of sulfate and phosphate salts; bases; zeolites or modified zeolites; metal oxides or modified metal oxides; supercritical water, or mixtures thereof.

### IV Process for the Production of Acrylic Acid or its Derivatives from Hydroxypropionic Acid or its Derivatives

A process for converting hydroxypropionic acid, hydroxypropionic acid derivatives, or mixtures thereof to acrylic acid, acrylic acid derivatives, or mixtures thereof of the present invention comprises the following steps: a) providing an aqueous solution comprising hydroxypropionic acid, hydroxypropionic acid derivatives, or mixtures thereof, wherein said hydroxypropionic acid is in monomeric form in the aqueous solution; b) combining the aqueous solution with an inert gas to form an aqueous solution / gas blend; c) evaporating the aqueous solution gas / blend to produce a gaseous mixture; and d) dehydrating the gaseous mixture by contacting the mixture with a dehydration catalyst under a pressure of at least about 80 psig.

Hydroxypropionic acid can be 3-hydroxypropionic acid, 2-hydroxypropionic acid (also called, lactic acid), 2-methyl hydroxypropionic acid, or mixtures thereof. Derivatives of hydroxypropionic acid can be metal or ammonium salts of hydroxypropionic acid, alkyl esters of hydroxypropionic acid, alkyl esters of 2-methyl hydroxypropionic acid, cyclic di-esters of hydroxypropionic acid, hydroxypropionic acid anhydride, or a mixture thereof. Non-limiting examples of metal salts of hydroxypropionic acid are sodium hydroxypropionate, potassium hydroxypropionate, and calcium hydroxypropionate. Non-limiting examples of alkyl esters of hydroxypropionic acid are methyl hydroxypropionate, ethyl hydroxypropionate, butyl hydroxypropionate, 2-ethylhexyl hydroxypropionate, or mixtures thereof. A non-limiting example of cyclic di-esters of hydroxypropionic acid is dilactide.

Hydroxypropionic acid can be in monomeric form or as oligomers in an aqueous solution of hydroxypropionic acid, hydroxypropionic acid derivatives, or mixtures thereof. The oligomers of the hydroxypropionic acid in an aqueous solution of hydroxypropionic acid, hydroxypropionic acid derivatives, or mixtures thereof are less than about 25 wt% based on the total amount of hydroxypropionic acid, hydroxypropionic acid derivatives, or mixtures thereof. The oligomers of the hydroxypropionic acid in an aqueous solution of hydroxypropionic acid, hydroxypropionic acid derivatives, or mixtures thereof are less than about 10 wt% based on the total amount of hydroxypropionic acid, hydroxypropionic acid derivatives, or mixtures thereof. The oligomers of the hydroxypropionic acid in an aqueous solution of hydroxypropionic acid, hydroxypropionic acid derivatives, or mixtures thereof are less than about 5 wt% based on the total amount of hydroxypropionic acid, hydroxypropionic acid derivatives, or mixtures thereof. The hydroxypropionic acid is in monomeric form in an aqueous solution of hydroxypropionic acid, hydroxypropionic acid derivatives, or mixtures thereof. The process steps to remove the oligomers from the aqueous solution can be purification or diluting with water and heating. The heating step can involve heating the aqueous solution of hydroxypropionic acid, hydroxypropionic acid derivatives, or mixtures thereof at a temperature from about 50°C to about 100°C to remove the oligomers of the hydroxypropionic acid. The heating step can involve heating the lactic acid aqueous solution at a temperature from about 95°C to about 100°C to remove the oligomers of the lactic acid and produce a monomeric lactic acid aqueous solution comprising at least 95 wt% of lactic acid in monomeric form based on the total amount of lactic acid. 88 wt% lactic acid aqueous solution (e.g. from Purac Corp., Lincolnshire, IL) is diluted with water to form an about 20 wt% lactic acid aqueous solution, to remove the ester impurities that are produced from the intermolecular condensation reaction. These esters can result in loss of product due to their high boiling point and oligomerization in the evaporation stage of the process. Additionally, these esters can cause coking, catalyst deactivation, and reactor plugging. As the water content decreases in the aqueous solution, the loss of feed material to the catalytic reaction, due to losses in the evaporation step, increases.

The hydroxypropionic acid is lactic acid or 2-methyl lactic acid. The hydroxypropionic acid is lactic acid. Lactic acid can be L-lactic acid, D-lactic acid, or mixtures thereof. The hydroxypropionic acid derivative is methyl lactate. Methyl lactate can be neat or in an aqueous solution.

Acrylic acid derivatives can be metal or ammonium salts of acrylic acid, alkyl esters of acrylic acid, acrylic acid oligomers, or a mixture thereof. Non-limiting examples of metal salts of acrylic acid are sodium acrylate, potassium acrylate, and calcium acrylate. Non-limiting examples of alkyl esters of acrylic acid are methyl acrylate, ethyl acrylate, butyl acrylate, 2-ethylhexyl acrylate, or mixtures thereof.

The concentration of the hydroxypropionic acid, hydroxypropionic acid derivatives, or mixtures thereof in the aqueous solution is between about 5 wt% and about 50 wt%. The concentration of the hydroxypropionic acid, hydroxypropionic acid derivatives, or mixtures thereof in the aqueous solution is between about 10 wt% and about 25 wt%. The concentration of the hydroxypropionic acid, hydroxypropionic acid derivatives, or mixtures thereof in the aqueous solution is about 20 wt%.

The aqueous solution can be combined with an inert gas to form an aqueous solution / gas blend. Non-limiting examples of the inert gas are air, nitrogen, helium, argon, carbon dioxide, carbon monoxide, steam, and mixtures thereof. The inert gas can be introduced to the evaporating step separately or in combination with the aqueous solution. The aqueous solution can be introduced with a simple tube or through atomization nozzles. Non-limiting examples of atomization nozzles include fan nozzles, pressure swirl atomizers, air blast atomizers, two-fluid atomizers, rotary atomizers, and supercritical carbon dioxide atomizers. The droplets of the aqueous solution are less than about 500 µm in diameter. The droplets of the aqueous solution are less than about 200 µm in diameter. The droplets of the aqueous solution are less than about 100 µm in diameter. In the evaporating step, the aqueous solution / gas blend is heated to give a gaseous mixture.

The temperature during the evaporating step is from about 165°C to about 450°C. The temperature during the evaporating step is from about 250°C to about 375°C. The gas hourly space velocity (GHSV) in the evaporating step is from about 720 h⁻¹ to 3,600 h⁻¹. The gas hourly space velocity (GHSV) in the evaporating step is about 7,200 h⁻¹. The evaporating step can be performed at either atmospheric pressure or higher pressure. The evaporating step is performed under a pressure from about 80 psig to about 550 psig. The evaporating step is performed under a pressure from about 300 psig to about 400 psig. The evaporating step is performed under a pressure from about 350 psig to about 375 psig. The gaseous mixture comprises from about 0.5 mol% to about 50 mol% hydroxypropionic acid, hydroxypropionic acid derivatives, or mixtures thereof. The gaseous mixture comprises from about 1 mol% to about 10 mol% hydroxypropionic acid, hydroxypropionic acid derivatives, or mixtures thereof. gaseous mixture comprises from about 1.5 mol% to about 3.5 mol% hydroxypropionic acid, hydroxypropionic acid derivatives, or mixtures thereof. The gaseous mixture comprises about 2.5 mol% hydroxypropionic acid, hydroxypropionic acid derivatives, or mixtures thereof.

The evaporating step can be performed in various types of equipment, such as, but not limited to, plate heat exchanger, empty flow reactor, and fixed bed flow reactor. Regardless of the type of the reactor, the reactor has an interior surface comprising material selected from the group consisting of quartz, borosilicate glass, silicon, hastelloy, inconel, manufactured sapphire, stainless steel, and mixtures thereof. The reactor has an interior surface comprising material selected from the group consisting of quartz, borosilicate glass, and mixtures thereof. The evaporating step can be performed in a reactor with the aqueous solution flowing down, or flowing up, or flowing horizontally. The evaporating step is performed in a reactor with the aqueous solution flowing down. Also, the evaporating step can be done in a batch form.

The gaseous mixture from the evaporating step is converted to acrylic acid, acrylic acid derivatives, and mixture thereof by contact it with a dehydration catalyst in the dehydrating step. The dehydration catalyst can be selected from the group comprising sulfates, phosphates, metal oxides, aluminates, silicates, aluminosilicates (e.g., zeolites), arsenates, nitrates, vanadates, niobates, tantalates, selenates, arsenatophosphates, phosphoaluminates, phosphoborates, phosphocromates, phosphomolybdates, phosphosilicates, phosphosulfates, phosphotungstates, and mixtures thereof, and others that may be apparent to those having ordinary skill in the art. The catalyst can contain an inert support that is constructed of a material comprising silicates, aluminates, carbons, metal oxides, and mixtures thereof. The dehydrating step is performed in a reactor, wherein the reactor has an interior surface comprising material selected from the group consisting of quartz, borosilicate glass, silicon, hastelloy, inconel, manufactured sapphire, stainless steel, and mixtures thereof. The the dehydrating step is performed in a reactor, wherein the reactor has an interior surface comprising material selected from the group consisting of quartz, borosilicate glass, and mixtures thereof. The temperature during the dehydrating step is from about 150°C to about 500°C. The temperature during the dehydrating step is from about 300°C to about 450°C. The GHSV in the dehydrating step is from about 720 h⁻¹ to about 36,000 h⁻¹. The GHSV in the dehydrating step is about 3,600 h⁻¹. The dehydrating step can be performed at higher than atmospheric pressure. The the dehydrating step is performed under a pressure of at least about 80 psig. The dehydrating step is performed under a pressure from about 80 psig to about 550 psig. The dehydrating step is performed under a pressure from about 150 psig to about 500 psig. The dehydrating step is performed under a pressure from about 300 psig to about 400 psig. The dehydrating step can be performed in a reactor with the gaseous mixture flowing down, flowing up, or flowing horizontally. The dehydrating step is performed in a reactor with the gaseous mixture flowing down. Also, the dehydrating step can be done in a batch form.

The evaporating and dehydrating steps are combined in a single step. The evaporating and dehydrating steps are performed sequentially in a single reactor. The evaporating and dehydrating steps are performed sequentially in a tandem reactor.

The selectivity of acrylic acid, acrylic acid derivatives, and mixture thereof from hydroxypropionic acid, hydroxypropionic acid derivatives, or mixtures thereof is at least about 50%. The selectivity of acrylic acid, acrylic acid derivatives, and mixture thereof from hydroxypropionic acid, hydroxypropionic acid derivatives, or mixtures thereof is at least about 80%. The selectivity of propanoic acid from hydroxypropionic acid, hydroxypropionic acid derivatives, or mixtures thereof is less than about 5%. The selectivity of propanoic acid from hydroxypropionic acid, hydroxypropionic acid derivatives, or mixtures thereof is less than about 1%. The conversion of the hydroxypropionic acid, hydroxypropionic acid derivatives, or mixtures thereof is more than about 50%. The conversion of the hydroxypropionic acid, hydroxypropionic acid derivatives, or mixtures thereof is more than about 80%.

### V Examples

The following examples are provided to illustrate the invention, but are not intended to limit the scope thereof.

### EXAMPLE 1

Solid dibasic potassium phosphate, K₂HPO₄ (36.40 g, 209 mmol, ≥ 98%; Sigma - Aldrich Co., St. Louis, MO; catalog # P3786) was mixed quickly with an aqueous solution of barium nitrate, Ba(NO₃)₂ (2050 mL of a 0.08 g/mL stock solution, 627 mmol, 99.999%; Sigma - Aldrich Co., St. Louis, MO; catalog # 202754) at room temperature. Phosphoric acid, H₃PO₄ (58.7 mL of an 85 wt%, density = 1.684 g/mL, 857 mmol; Acros Organics, Geel, Belgium; catalog # 295700010), was added to the slurry, providing a solution containing potassium (K⁺, M^{I}) and barium (Ba²⁺, M^{II}) cations. The final pH of the suspension was about 1.6. The acid-containing suspension was then dried slowly in a glass beaker at 80°C using a heating plate while magnetically stirring the suspension until the liquid was evaporated and the material was almost completely dried. Heating was continued in a oven with air circulation (G1530A, HP6890 GC; Agilent Corp., Santa Clara, CA) at 50°C for 5.3 h, then at 80°C for 10 h (0.5°C/min ramp), following by cooling down at 25°C. The material was calcined at 120°C for 2 hours (0.5°C/min ramp) followed by 450°C for 4 hours (2°C/min ramp) using the same oven. After calcination, the material was left inside the oven until it cooled down by itself at a temperature below 25°C before it was taken out of the oven. Finally, the catalyst was ground and sieved to about 100 µm to about 200 µm.

### EXAMPLE 2

454 g of an 88 wt% L-lactic acid solution (Purac Corp., Lincolnshire, IL) was diluted with 1,300 g of water. The diluted solution was heated to 95°C and held at that temperature with stirring for about 4 to 12 hours. Then, the solution was cooled to room temperature, and its lactic acid and lactic acid oligomers concentrations were measured by HPLC (Agilent 1100 system; Santa Clara, CA) equipped with a DAD detector and a Waters Atlantis T3 column (Catalog # 186003748; Milford, MA) using methods generally known by those having ordinary skill in the art. The solution was essentially free of oligomers. Finally, the solution was further diluted with water to yield a 20 wt% L-lactic acid aqueous solution and essentially free of oligomers.

### EXAMPLE 3

The reactor consisted of an electric clam shell furnace (Applied Test systems, Butler, PA) with an 8" (20.3 cm) heated zone with one temperature controller connected in series to another electric clam shell furnace (Applied Test Systems, Butler, PA) with a 16" (40.6 cm) heated zone containing two temperature controllers and a reactor tube. The reactor tube consisted of a 13" (33 cm) borosilicate glass-lined tube (SGE Analytical Science Pty Ltd., Ringwood, Australia)) and a 23" (58.4 cm) borosilicate glass lined tube connected in series using a Swagelok™ tee fitting equipped with an internal thermocouple and having an inside diameter of 9.5 mm. The head of the column was fitted with a 1/8" (3.2 mm) stainless steel nitrogen feed line and a 1/16" (1.6 mm) fused silica lined stainless steel liquid feed supply line connected to a HPLC pump (Smartline 100, Knauer, Berlin, Germany) that was connected to a lactic acid feed tank. The bottom of the reactor was connected to a Teflon-lined catch tank using 1/8" (3.2 mm) fused silica lined stainless steel tubing and Swagelok™ fittings. The reactor column was packed with a plug of glass wool, 13 g of fused quartz, 16" (40.7 cm) with catalyst of Example 1 (47 g and 28.8 mL packed bed volume) and topped with 25 g of fused quartz. The reactor tube was placed in an aluminum block and placed into the reactor from above in a downward flow. The reactor was preheated to 375°C overnight under 0.25 L/min nitrogen. The nitrogen feed was increased to 0.85 L/min during the experiment. The liquid feed was a 20 wt% aqueous solution of L-lactic acid, prepared as in Example 2, and fed at 0.845 mL/min (LHSV of 1.8 h⁻¹; 50.7 g/h), giving a residence time of about 1 s (GHSV of 3,600 h⁻¹) at STP conditions. The clam shell heaters were adjusted to give an internal temperature about 350°C. After flowing through the reactor, the gaseous stream was cooled and the liquid was collected in the catch tank for analysis by off-line HPLC using an Agilent 1100 system (Santa Clara, CA) equipped with a DAD detector and a Waters Atlantis T3 column (Catalog # 186003748; Milford, MA) using methods generally known by those having ordinary skill in the art. The gaseous stream was analyzed on-line by GC using an Agilent 7890 system (Santa Clara, CA) equipped with a FID detector and Varian CP-Para Bond Q column (Catalog # CP7351; Santa Clara, CA). The crude reaction mixture was cooled and collected over 159 h to give 748 g acrylic acid as a crude mixture in 54% yield, 75% acrylic acid selectivity, and 69% conversion of lactic acid. The acrylic acid yield, corrected for the losses during the evaporating step, was 61% and its selectivity was 89%. The acrylic acid aqueous concentration was 8.4 wt%, and that of lactic acid was 6.3 wt%.

### EXAMPLE 4

The reaction mixtures from Example 3 were combined into four batches and isolated to give an acrylic acid solution of 668.9 g of acrylic acid in water. A stabilizer (200 - 400 ppm phenothiazine) was added to each batch and the batches were extracted with ethyl acetate several times. The combined ethyl acetate layers were dried with sodium sulfate, treated with activated carbon, filtered over diatomaceous earth, and washed with ethyl acetate. The filtrate was evaporated at 40 - 70 mm Hg with a bath temperature of 23°C - 40°C to give bio-based acrylic acid as a pale yellow liquid (81.4% yield). The bio-based acrylic acid was then fractionally distilled at 40 mm Hg using a 12 inch 14/20 Vigreux column. The product was collected with head temperature of 59°C - 62°C, stabilized with 4-methoxy phenol, and placed in a 3°C - 5°C fridge overnight. The solution was removed from the fridge and thawed. The resulting liquid was decanted off and the solids were combined. The crystallization was repeated several times. The four batches were combined to give glacial acrylic acid (218 g, 32.6% yield on purification). The glacial acrylic acid composition consisted of 99.1 wt% acrylic acid, 0.1 wt% water, 0.7 wt% propanoic acid, and 0.1 wt% lactic acid.

### EXAMPLE 5

The bio-based content of the glacial acrylic acid composition of Example 4 is measured in accordance with ASTM D6866 Method B, as described in the Test and Calculation Procedures section below, and is greater than about 90%.

### VII Test and Calculation Procedures

The bio-based content of a material is measured using the ASTM D6866 method, which allows the determination of the bio-based content of materials using radiocarbon analysis by accelerator mass spectrometry, liquid scintillation counting, and isotope mass spectrometry. When nitrogen in the atmosphere is struck by an ultraviolet light produced neutron, it loses a proton and forms carbon that has a molecular weight of 14, which is radioactive. This ¹⁴C is immediately oxidized into carbon dioxide, which represents a small, but measurable fraction of atmospheric carbon. Atmospheric carbon dioxide is cycled by green plants to make organic molecules during the process known as photosynthesis. The cycle is completed when the green plants or other forms of life metabolize the organic molecules producing carbon dioxide, which causes the release of carbon dioxide back to the atmosphere. Virtually all forms of life on Earth depend on this green plant production of organic molecules to produce the chemical energy that facilitates growth and reproduction. Therefore, the ¹⁴C that exists in the atmosphere becomes part of all life forms and their biological products. These renewably based organic molecules that biodegrade to carbon dioxide do not contribute to global warming because no net increase of carbon is emitted to the atmosphere. In contrast, fossil fuel-based carbon does not have the signature radiocarbon ratio of atmospheric carbon dioxide. *See* WO 2009/155086, incorporated herein by reference.

The application of ASTM D6866 to derive a "bio-based content" is built on the same concepts as radiocarbon dating, but without use of the age equations. The analysis is performed by deriving a ratio of the amount of radiocarbon (¹⁴C) in an unknown sample to that of a modern reference standard. The ratio is reported as a percentage with the units "pMC" (percent modern carbon). If the material being analyzed is a mixture of present day radiocarbon and fossil carbon (containing no radiocarbon), then the pMC value obtained correlates directly to the amount of biomass material present in the sample. The modern reference standard used in radiocarbon dating is a NIST (National Institute of Standards and Technology) standard with a known radiocarbon content equivalent approximately to the year AD 1950. The year AD 1950 was chosen because it represented a time prior to thermo-nuclear weapons testing, which introduced large amounts of excess radiocarbon into the atmosphere with each explosion (termed "bomb carbon"). The AD 1950 reference represents 100 pMC. "Bomb carbon" in the atmosphere reached almost twice normal levels in 1963 at the peak of testing and prior to the treaty halting the testing. Its distribution within the atmosphere has been approximated since its appearance, showing values that are greater than 100 pMC for plants and animals living since AD 1950. The distribution of bomb carbon has gradually decreased over time, with today's value being near 107.5 pMC. As a result, a fresh biomass material, such as corn, could result in a radiocarbon signature near 107.5 pMC.

Petroleum-based carbon does not have the signature radiocarbon ratio of atmospheric carbon dioxide. Research has noted that fossil fuels and petrochemicals have less than about 1 pMC, and typically less than about 0.1 pMC, for example, less than about 0.03 pMC. However, compounds derived entirely from renewable resources have at least about 95 percent modern carbon (pMC), and may have at least about 99 pMC, including about 100 pMC.

Combining fossil carbon with present day carbon into a material will result in a dilution of the present day pMC content. By presuming that 107.5 pMC represents present day biomass materials and 0 pMC represents petroleum derivatives, the measured pMC value for that material will reflect the proportions of the two component types. A material derived 100% from present day soybeans would give a radiocarbon signature near 107.5 pMC. If that material was diluted with 50% petroleum derivatives, it would give a radiocarbon signature near 54 pMC.

A bio-based content result is derived by assigning 100% equal to 107.5 pMC and 0% equal to 0 pMC. In this regard, a sample measuring 99 pMC will give an equivalent bio-based content result of 93%.

Assessment of the materials described herein was done in accordance with ASTM D6866, particularly with Method B. The mean values encompass an absolute range of 6% (plus and minus 3% on either side of the bio-based content value) to account for variations in end-component radiocarbon signatures. It is presumed that all materials are present day or fossil in origin and that the desired result is the amount of bio-component "present" in the material, not the amount of bio-material "used" in the manufacturing process.

Other techniques for assessing the bio-based content of materials are described in U.S. Patent Nos. 3,885155, 4,427,884, 4,973,841, 5,438,194, and 5,661,299, and WO 2009/155086, each incorporated herein by reference.

For example, acrylic acid contains three carbon atoms in its structural unit. If acrylic acid is derived from a renewable resource, then it theoretically has a bio-based content of 100%, because all of the carbon atoms are derived from a renewable resource.

The foregoing description is given for clearness of understanding only, and no unnecessary limitations should be understood therefrom, as modifications within the scope of the invention may be apparent to those having ordinary skill in the art.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. A glacial acrylic acid composition comprising at least 98 wt% acrylic acid, and wherein
a portion of the remaining impurities in said glacial acrylic acid composition is propanoic acid;
wherein said glacial acrylic acid composition has a bio-based content greater than 3%; and
wherein the bio-based content is the amount of carbon from a renewable resource in a material as a percent of the weight (mass) of the total organic carbon in the material, as determined by ASTM D6866-10, Method B.

2. The glacial acrylic acid composition of Claim 1 wherein said glacial acrylic acid composition has a bio-based content greater than 30%.

3. The glacial acrylic acid composition of Claim 1 wherein said glacial acrylic acid composition has a bio-based content greater than 90%.

4. A process of producing a glacial acrylic acid composition comprising:
a. Providing an aqueous solution of acrylic acid comprising: 1) acrylic acid; and 2) lactic acid, lactic acid derivatives, or mixtures thereof, and wherein said aqueous solution of acrylic acid is essentially free of maleic anhydride, furfural, and formic acid;
b. Extracting said aqueous solution of acrylic acid, with a solvent to produce an extract;
c. Drying said extract to produce a dried extract;
d. Distilling said dried extract to produce distilled acrylic acid composition;
e. Cooling said distilled acrylic acid composition to a temperature from -21 °C to 14°C to produce crystals of acrylic acid;
f. Partially melting said crystals of acrylic acid to produce a liquid / solid mixture;
g. Decanting said liquid / solid mixture to produce a purified acrylic acid solid composition;
h. Fully melting said purified acrylic acid solid composition to produce a purified acrylic acid liquid composition; and
i. Determining the acrylic acid purity of said purified acrylic acid liquid composition, and if the purity is less than 98 wt% acrylic acid, repeating said cooling, partially melting, decanting, and fully melting steps on the purified acrylic acid liquid composition until a purity of 98 wt% acrylic acid is achieved and said glacial acrylic acid composition is produced; wherein the produced glacial acrylic acid composition comprises at least 98 wt% acrylic acid, and wherein a portion of the remaining impurities in said glacial acrylic acid composition is propanoic acid;
wherein said glacial acrylic acid composition has a bio-based content greater than 3%; and
wherein the bio-based content is the amount of carbon from a renewable resource in a material as a percent of the weight (mass) of the total organic carbon in the material, as determined by ASTM D6866-10, Method B.

5. The process of claim 4, wherein the aqueous solution of acrylic acid comprises from 4 wt% to 80 wt% acrylic acid.

6. The process of claim 4 or 5, wherein the aqueous solution of acrylic acid comprises from 5 wt% to 25 wt% acrylic acid.

7. The process of any of claims 4 to 6, wherein the aqueous solution of acrylic acid comprises from 0.001 wt% to 50 wt% lactic acid, lactic acid derivatives, or mixtures thereof.

8. The process of any of claims 4 to 7, wherein the aqueous solution of acrylic acid comprises from 0.001 wt% to 20 wt% lactic acid, lactic acid derivatives, or mixtures thereof.

9. The process of any of claims 4 to 8, wherein said solvent is selected from the group consisting of ethyl acetate, isobutyl acetate, methyl acetate, toluene, dimethyl phthalate, hexane, pentane, diphenyl ether, ethyl hexanoic acid, N-methylpyrrolidone, C6 to C10 paraffin fractions, and mixtures thereof.

10. The process of any of claims 4 to 9, wherein said drying is performed by azeotropic distillation.

11. The process of any of claims 4 to 10, wherein said drying is performed by distillation.

12. The process of any of claims 4 to 11, wherein said drying is performed by sorption.

13. The process of any of claims 4 to 12, wherein said sorption is performed on a solid powder selected from the group consisting of magnesium sulfate, sodium sulfate, calcium sulfate, molecular sieves, metal hydrides, reactive metals, and mixtures thereof.

## Patentansprüche

1. Eisacrylsäurezusammensetzung, die mindestens 98 Gew.-% Acrylsäure umfasst und wobei ein Teil der verbleibenden Fremdstoffe in der Eisacrylsäurezusammensetzung Propansäure ist; wobei die Eisacrylsäurezusammensetzung einen biobasierten Gehalt von mehr als 3 % aufweist; und
wobei der biobasierte Gehalt die Menge an Kohlenstoff aus einer erneuerbaren Ressource in einem Material in Gewichtsprozent (Massenprozent) des gesamten organischen Kohlenstoffs in dem Material ist, wie durch ASTM D6866-10, Methode B, bestimmt.

2. Eisacrylsäurezusammensetzung nach Anspruch 1, wobei die Eisacrylsäurezusammensetzung einen biobasierten Gehalt von mehr als 30 % aufweist.

3. Eisacrylsäurezusammensetzung nach Anspruch 1, wobei die Eisacrylsäurezusammensetzung einen biobasierten Gehalt von mehr als 90 % aufweist.

4. Verfahren zur Herstellung einer Eisacrylsäurezusammensetzung, umfassend:
a. Bereitstellen einer wässrigen Lösung von Acrylsäure, umfassend: 1) Acrylsäure; und 2) Milchsäure, Milchsäurederivate oder Mischungen davon, und wobei die wässrige Lösung von Acrylsäure im Wesentlichen frei von Maleinsäureanhydrid, Furfural und Ameisensäure ist;
b. Extrahieren der wässrigen Lösung von Acrylsäure mit einem Lösungsmittel, um einen Extrakt herzustellen;
c. Trocknen des Extrakts, um einen getrockneten Extrakt herzustellen;
d. Destillieren des getrockneten Extrakts, um eine destillierte Acrylsäurezusammensetzung herzustellen;
e. Abkühlen der destillierten Acrylsäurezusammensetzung auf eine Temperatur von -21 °C bis 14 °C, um Kristalle von Acrylsäure herzustellen;
f. Teilweises Schmelzen der Kristalle von Acrylsäure, um eine Flüssigkeit/Feststoff-Mischung herzustellen;
g. Dekantieren der Flüssigkeit/Feststoff-Mischung, um eine gereinigte feste Acrylsäurezusammensetzung herzustellen;
h. Vollständiges Schmelzen der gereinigten festen Acrylsäurezusammensetzung, um eine gereinigte flüssige Acrylsäurezusammensetzung herzustellen; und
i. Bestimmen der Acrylsäurereinheit der gereinigten flüssigen Acrylsäurezusammensetzung, und wenn die Reinheit weniger als 98 Gew.-% Acrylsäure beträgt, Wiederholen der Schritte des Abkühlens, teilweisen Schmelzens, Dekantierens und vollständigen Schmelzens an der gereinigten flüssigen Acrylsäurezusammensetzung, bis eine Reinheit von 98 Gew.-% Acrylsäure erreicht ist und die Eisacrylsäurezusammensetzung hergestellt ist;
wobei die hergestellte Eisacrylsäurezusammensetzung mindestens 98 Gew.-% Acrylsäure umfasst und wobei ein Teil der verbleibenden Fremdstoffe in der Eisacrylsäurezusammensetzung Propansäure ist;
wobei die Eisacrylsäurezusammensetzung einen biobasierten Gehalt von mehr als 3 % aufweist; und
wobei der biobasierte Gehalt die Menge an Kohlenstoff aus einer erneuerbaren Ressource in einem Material in Gewichtsprozent (Massenprozent) des gesamten organischen Kohlenstoffs in dem Material ist, wie durch ASTM D6866-10, Methode B, bestimmt.

5. Verfahren nach Anspruch 4, wobei die wässrige Lösung von Acrylsäure 4 Gew.-% bis 80 Gew.-% Acrylsäure umfasst.

6. Verfahren nach Anspruch 4 oder 5, wobei die wässrige Lösung von Acrylsäure 5 Gew.-% bis 25 Gew.-% Acrylsäure umfasst.

7. Verfahren nach einem der Ansprüche 4 bis 6, wobei die wässrige Lösung von Acrylsäure 0,001 Gew.-% bis 50 Gew.-% Milchsäure, Milchsäurederivate oder Mischungen davon umfasst.

8. Verfahren nach einem der Ansprüche 4 bis 7, wobei die wässrige Lösung von Acrylsäure 0,001 Gew.-% bis 20 Gew.-% Milchsäure, Milchsäurederivate oder Mischungen davon umfasst.

9. Verfahren nach einem der Ansprüche 4 bis 8, wobei das Lösungsmittel ausgewählt ist aus der Gruppe, bestehend aus Ethylacetat, Isobutylacetat, Methylacetat, Toluol, Dimethylphthalat, Hexan, Pentan, Diphenylether, Ethylhexansäure, N-Methylyrrolidon, C₆- bis C₁₀-Paraffinfraktionen und Mischungen davon.

10. Verfahren nach einem der Ansprüche 4 bis 9, wobei das Trocknen durch azeotrope Destillation durchgeführt wird.

11. Verfahren nach einem der Ansprüche 4 bis 10, wobei das Trocknen durch Destillation durchgeführt wird.

12. Verfahren nach einem der Ansprüche 4 bis 11, wobei das Trocknen durch Sorption durchgeführt wird.

13. Verfahren nach einem der Ansprüche 4 bis 12, wobei die Sorption an einem festen Pulver durchgeführt wird, das ausgewählt ist aus der Gruppe, bestehend aus Magnesiumsulfat, Natriumsulfat, Calciumsulfat, Molekularsieben, Metallhydriden, reaktiven Metallen und Mischungen davon.

## Revendications

1. Composition d'acide acrylique glacial comprenant au moins 98 % en poids d'acide acrylique, et dans laquelle une partie des impuretés restantes dans ladite composition d'acide acrylique glacial est de l'acide propanoïque ; dans laquelle ladite composition d'acide acrylique glacial a une teneur d'origine biologique supérieure à 3 % ; et
dans laquelle la teneur d'origine biologique est la quantité de carbone provenant d'une ressource renouvelable dans un matériau en tant que pour cent du poids (de la masse) du carbone organique total dans le matériau, telle que déterminée par ASTM D6866-10, Procédé B.

2. Composition d'acide acrylique glacial selon la revendication 1 dans laquelle ladite composition d'acide acrylique glacial a une teneur d'origine biologique supérieure à 30 %.

3. Composition d'acide acrylique glacial selon la revendication 1 dans laquelle ladite composition d'acide acrylique glacial a une teneur d'origine biologique supérieure à 90 %.

4. Processus de production d'une composition d'acide acrylique glacial comprenant :
a. la fourniture d'une solution aqueuse d'acide acrylique comprenant : 1) de l'acide acrylique ; et 2) de l'acide lactique, des dérivés d'acide lactique, ou des mélanges de ceux-ci, et dans lequel ladite solution aqueuse d'acide acrylique est pratiquement exempte d'anhydride maléique, de furfural, et d'acide formique ;
b. l'extraction de ladite solution aqueuse d'acide acrylique, avec un solvant pour produire un extrait ;
c. le séchage dudit extrait pour produire un extrait séché ;
d. la distillation dudit extrait séché pour produire une composition d'acide acrylique distillé ;
e. le refroidissement de ladite composition d'acide acrylique distillé à une température allant de -21 °C à 14 °C pour produire des cristaux d'acide acrylique ;
f. la fusion partielle desdits cristaux d'acide acrylique pour produire un mélange liquide/solide ;
g. la décantation dudit mélange liquide/solide pour produire une composition solide d'acide acrylique purifié ;
h. la fusion complète de ladite composition solide d'acide acrylique purifié pour produire une composition liquide d'acide acrylique purifié ; et
i. la détermination de la pureté d'acide acrylique de ladite composition liquide d'acide acrylique purifié, et si la pureté est inférieure à 98 % en poids d'acide acrylique, la répétition desdites étapes de refroidissement, de fusion partielle, de décantation, et de fusion complète sur la composition liquide d'acide acrylique purifié jusqu'à ce qu'une pureté de 98 % en poids d'acide acrylique soit obtenue et que ladite composition d'acide acrylique glacial soit produite ;
dans lequel la composition d'acide acrylique glacial produite comprend au moins 98 % en poids d'acide acrylique, et dans lequel une partie des impuretés restantes dans ladite composition d'acide acrylique glacial est de l'acide propanoïque ;
dans laquelle ladite composition d'acide acrylique glacial a une teneur d'origine biologique supérieure à 3 % ; et
dans laquelle la teneur d'origine biologique est la quantité de carbone provenant d'une ressource renouvelable dans un matériau en tant que pour cent du poids (de la masse) du carbone organique total dans le matériau, telle que déterminée par ASTM D6866-10, Procédé B.

5. Processus selon la revendication 4, dans lequel la solution aqueuse d'acide acrylique comprend de 4 % en poids à 80 % en poids d'acide acrylique.

6. Processus selon la revendication 4 ou 5, dans lequel la solution aqueuse d'acide acrylique comprend de 5 % en poids à 25 % en poids d'acide acrylique.

7. Processus selon l'une quelconque des revendications 4 à 6, dans lequel la solution aqueuse d'acide acrylique comprend de 0,001 % en poids à 50 % en poids d'acide lactique, de dérivés d'acide lactique, ou de mélanges de ceux-ci.

8. Processus selon l'une quelconque des revendications 4 à 7, dans lequel la solution aqueuse d'acide acrylique comprend de 0,001 % en poids à 20 % en poids d'acide lactique, de dérivés d'acide lactique, ou de mélanges de ceux-ci.

9. Processus selon l'une quelconque des revendications 4 à 8, dans lequel ledit solvant est choisi dans le groupe constitué d'acétate d'éthyle, acétate d'isobutyle, acétate de méthyle, toluène, phtalate de diméthyle, hexane, pentane, éther diphénylique, acide éthyl-hexanoïque, N-méthylpyrrolidone, fractions de paraffine en C6 à C10, et des mélanges de ceux-ci.

10. Processus selon l'une quelconque des revendications 4 à 9, dans lequel ledit séchage est mis en œuvre par distillation azéotrope.

11. Processus selon l'une quelconque des revendications 4 à 10, dans lequel ledit séchage est mis en œuvre par distillation.

12. Processus selon l'une quelconque des revendications 4 à 11, dans lequel ledit séchage est mis en œuvre par sorption.

13. Processus selon l'une quelconque des revendications 4 à 12, dans lequel ladite sorption est mise en œuvre sur une poudre solide choisie dans le groupe constitué de sulfate de magnésium, sulfate de sodium, sulfate de calcium, tamis moléculaire, hydrures métalliques, métaux réactifs, et des mélanges de ceux-ci.
